# EUROPÄISCHE PATENTANMELDUNG

(11) **EP 3 366 332 A1**
(43) Veröffentlichungstag der Anmeldung: **29.08.2018**
(21) Anmeldenummer: 17157481.7
(22) Anmeldetag: 22.02.2017
(51) Int. Cl.: A61M 1/10, A61M 1/12

(54) **VORRICHTUNG UND VERFAHREN ZUR VERBINDUNG ZWEIER BLUTGEFÄSSABSCHNITTE**

(71) Anmelder: Berlin Heart GmbH, 12247 Berlin (DE)
(72) Erfinder: Menon, Ares K., 12209 Berlin (DE); Wöhling, Thomas, 21218 Seevetal (DE); Sandica, Eugen, 32547 Bad Oeynhausen (DE)
(74) Vertreter: Pfenning, Meinig & Partner mbB

(57) **Zusammenfassung**

Bei Patienten mit einer "Failing-Fontan"-Zirkulation ist ein häufig ein operativer Eingriff notwendig, um die Patienten vor einer Herztransplantation zu stabilisieren. Für eine derartige Stabilisierung wird ein System mit einer Vorrichtung vorgeschlagen, welche zwei Einflussöffnungen und eine Ausflussöffnung aufweist.

## Beschreibung

Gegenstand der vorliegenden Anmeldung ist eine Vorrichtung zur Verbindung zweier Blutgefäßabschnitte sowie eine Verwendung einer derartigen Vorrichtung und ein Verfahren zur Implantation einer derartigen Vorrichtung.

Bei der Behandlung angeborener Herzfehler erfordern sogenannte "Single-Ventricle"-Erkrankungen eine rasche Korrektur im Sinne einer Palliation. In der Medizin bezeichnet man mit "Single Ventricle" oder Ein-Kammer-Herz einen Formenkreis angeborener Herzfehlbildungen, bei denen sich während der fetalen Entwicklung lediglich eine funktionierende Herzkammer ausgebildet hat. Das Krankheitsbild umfasst vielfältige Formen, die auch unterschiedliche Auswirkungen auf den Kreislauf und das therapeutische Vorgehen haben. Gemeinsam ist allen Formen, dass wirklich nur eine Herzkammer existiert, oder eine Hauptkammer, die ihr Blut aus beiden Vorhöfen bekommt, mit einer Nebenkammer, die ihr Blut aus der Hauptkammer erhält. Man unterscheidet hierbei zwischen zwei Grundformen, je nachdem, ob sich die rechte Herzkammer oder die linke Herzkammer nicht hinreichend ausgebildet hat.

Die chirurgische Therapie in Form von Palliativeingriffen und einer Operation, die die Situation bessert, hängt von der genauen anatomischen Konstellation ab und wird für jeden Patienten, insbesondere für jedes Kind, individuell entschieden. Bei Fehlbildungen des rechten Ventrikels, vor allen Dingen bei Verschluss von Trikuspidal- und/oder Pulmonalklappe, wird eine Fontan-Operation gewählt. Bei Fehlbildungen des linken Ventrikels, insbesondere einem hypoplastischen Linksherz-Syndrom, wird eine Norwood-Operation vorgeschlagen.

Bei der Fontan-Operation wird in meist drei operativen Schritten das faktische Fehlen einer Herzkammer kompensiert durch die Etablierung einer einzelnen "Systemkammer", die den großen Körperkreislauf versorgt, und die Etablierung einer passiven Lungendurchblutung. Die einzig normal groß angelegte Herzkammer ist hier zumeist die rechte, eigentlich schwache Herzkammer. Diese muss den Körperkreislauf versorgen, während das Blut rein passiv über die großen Körpervenen (Hohlvenen) zurück in und durch die Lunge fließt, ohne dass eine Kammer aktiv dieses venöse Blut in die Lunge pumpt. Der finale Situs nennt sich "Fontan-Kreislauf" oder "totale cavo-pulmonale Anastomose" (auf Englisch: total cavo-pulmonary connection, kurz TCPC).

Typischerweise erleiden Patienten mit dieser Kreislaufform zwei grundsätzliche Probleme: Zum einen das Versagen der passiven Lungendurchblutung und/oder zum anderen das Versagen der dann einzigen "Systemherzkammer". Im ersten Fall kommt es wegen der fehlenden aktiven Pumpleistung zum venösen Rückstau in die untere Körperhälfte mit Wassereinlagerungen (Ödemen), Bauchwassersucht (Aszites), Stauung in Bauchorganen wie Leber, Niere und Darm mit entsprechend schweren Funktionsstörungen (Eiweißverlustsyndrom, Nieren- und Leberinsuffizienz etc.). Im zweiten Fall ermüdet - zumeist nach zwei bis drei Jahrzehnten - die Systemherzkammer, und es bildet sich eine Pumpschwäche aus. Der eigentlich schwache rechte Herzmuskel ist nicht kräftig genug ausgelegt für eine lebenslange Versorgung des großen Körperkreislaufs.

Eine Herztransplantation ist oft die einzig mögliche Therapieoption. Da aber der Ausgangszustand der Patienten mit einer sich verschlechternden Zirkulation (sogenannten "Failing Fontan") schlechter ist als bei den anderen Organempfängern, ist die Erfolgsrate nach einer Herztransplantation schlecht. Eines der Probleme ist die mangelnde systematische Konditionierung der Patienten mit Failing-Fontan-Zirkulation. Es fehlen Instrumente, die es ermöglichen, bei Failing-Fontan-Patienten eine Verbesserung des Grundzustands, d. h. eine Stabilisierung der Kreislaufsituation im Vorfeld einer Herztransplantation, zu erreichen.

Neben den bereits angesprochenen Herzfehlern kann die in dieser Anmeldung beschriebene Vorrichtung bzw. ein entsprechendes Verfahren zur Verwendung der Vorrichtung auch bei der Therapie anderer angeborener Herzfehler zur Anwendung kommen, wie beispielsweise Herzfehler, welche den zeitweisen Einsatz eines "Total Artificial Hearts" erfordern bis ein Spenderherz verfügbar oder einsetzbar ist.

Im Stand der Technik sind einige Techniken zur Verbesserung des Kreislaufs im Vorfeld einer Herztransplantation bekannt. In der US 2004/0147803 wird ein System mit einer aufblasbaren Manschette vorgestellt, wobei die Manschette um die Vena cava superior (englisch: superior vena cava, SVC) oder die Vena cava inferior (englisch: inferior vena cava, IVC) gelegt wird. Durch rhythmisches Aufblasen und Entlüften der Manschette wird die Vena cava stimuliert und eine zusätzliche Pumpleistung auf das Blut der Vena cava gebracht, so dass der Lungenkreislauf unterstützt wird.

In der EP 2 341956 A1 wird ein System vorgestellt, bei welchem eine Pumpe zur Unterstützung des Lungenkreislaufs direkt in die SVC oder IVC gelegt wird und im Bereich einer Fontan-Anastomose das Blut aktiv in die Lungenvenen pumpt.

Aufgabe der vorliegenden Anmeldung ist es, eine Alternative zu den oben genannten Vorrichtungen und Verfahren zur Verfügung zu stellen. Gelöst wird die Aufgabe nach den Maßgaben der vorliegenden Ansprüche.

Anmeldungsgemäß ist eine Vorrichtung zur Verbindung zweier Blutgefäßabschnitte, vorzugsweise des Endes der SVC und eines Endes der IVC, vorgesehen. Die Vorrichtung umfasst einen fluiddichten Hohlkörper mit einem ersten Endabschnitt, einem zweiten Endabschnitt und einem zwischen dem ersten und zweiten Endabschnitt angeordneten Zwischenabschnitt.

Der Hohlkörper schafft so eine direkte Verbindung zwischen den beiden Blutgefäßabschnitten und wird quasi als Prothese zwischen die beiden Blutgefäßabschnitte gesetzt. Dabei können der erste und/oder der zweite Endabschnitt so ausgebildet sein, dass sie direkt mit den Enden der jeweiligen Blutgefäßabschnitte verbunden werden. Alternativ kann an dem ersten und/oder zweiten Endabschnitt ein weiterer Materialabschnitt angeordnet werden, wobei der weitere Materialabschnitt mit dem Blutgefäß verbunden wird.

Der Hohlkörper ist fluiddicht, d. h., dass zwischen dem ersten und dem zweiten Endabschnitt hindurchströmende Flüssigkeit im Wesentlichen nur durch Öffnungen des Hohlkörpers austreten kann. Hierzu ist in dem ersten Endabschnitt eine erste Öffnung und in dem zweiten Endabschnitt eine zweite Öffnung vorgesehen. Somit wird vermieden, dass Flüssigkeit, wie beispielsweise Blut, durch den Hohlkörper bzw. eine Wand des Hohlkörpers nach außen dringen und sich in die Bauchhöhle ergießen kann.

Neben der ersten und der zweiten Öffnung weist der Zwischenabschnitt eine dritte Öffnung auf. Die dritte Öffnung dient zum Abfluss des durch den ersten und/oder den zweiten Endabschnitt einfließenden Blutes. Der Zwischenabschnitt weist dabei im Bereich der dritten Öffnung eine Kopplungsvorrichtung für eine Blutpumpe oder eine Blutkanüle, die mit einer Blutpumpe verbunden wird, auf.

Durch das Vorhandensein einer Kopplungsvorrichtung im Bereich der dritten Öffnung kann nach der Implantation der anmeldungsgemäßen Vorrichtung eine Blutpumpe entweder direkt mit der Kopplungsvorrichtung oder mit einer an der Kopplungsvorrichtung angeordneten Blutkanüle verbunden werden und so ein aktiver Pumpprozess des aus den beiden Blutgefäßabschnitten strömenden Blutes durch eine Pumpe unterstützt werden. Dabei kann die Vorrichtung Teil eines Systems sein, das neben der anmeldungsgemäßen Vorrichtung auch eine Blutpumpe zum Anschluss an die Vorrichtung umfasst. Bei der Blutpumpe kann es sich um eine extrakorporale oder eine intrakorporale Herzunterstützungspumpe handeln. Dabei wird die Kopplungsvorrichtung für die Blutpumpe oder die an die Kopplungsvorrichtung angeschlossene Blutkanüle direkt mit dem Einlass der Blutpumpe bzw. Herzunterstützungspumpe gekoppelt und der Auslass der Pumpe mittels einer weiteren Kanüle oder eines Grafts beispielsweise an die Lungenvenen angeschlossen.

Bei den Pumpen kann es sich beispielsweise um eine EXCOR®-Pumpe oder eine INCOR®-Pumpe der Berlin Heart GmbH, eine Pumpe der HeartMate-Serie von Thoratec, eine MVAD oder auch eine Kurzzeitherzpumpe, wie sie im Bereich von ECMO-Therapien eingesetzt wird, handeln. Auch sind weitere auf dem Markt erhältliche Herzunterstützungssysteme intrakorporaler oder extrakorporaler Natur in dem System anwendbar.

Im Wesentlichen wird durch die Vorrichtung ermöglicht, eine Blutpumpe zur Unterstützung des Lungenkreislaufs direkt an das Kreislaufsystem anzuschließen. Hierdurch wird die für den Patienten notwendige Entlastung des Kreislaufs sicher bewerkstelligt, und der Patient kann sich vor einer anstehenden Herztransplantation sicher erholen.

Weitere Ausführungsbeispiele finden sich in den abhängigen Ansprüchen und in den nachfolgenden Erläuterungen.

In einer Ausführungsform ist die Kopplungsvorrichtung integral mit dem Zwischenabschnitt verbunden. Dies bedeutet beispielsweise, dass die Kopplungsvorrichtung vorzugsweise einstückig mit dem Zwischenabschnitt hergestellt ist, d. h., dass der Zwischenabschnitt und die Kopplungsvorrichtung aus demselben Material bestehen. Hierdurch vereinfacht sich die Handhabung der Vorrichtung beim Anbinden der Vorrichtung an eine Blutpumpe. Alternativ wird unter "integral" jedoch verstanden, dass die Kopplungsvorrichtung zwar nicht einstückig, aber fest und dauerhaft mit dem Zwischenabschnitt verbunden ist. So kann beispielsweise die Kopplungsvorrichtung mit dem Zwischenabschnitt vernäht, verschweißt oder verklebt sein. Bei einigen Ausführungsformen scheint es einfacher, die Kopplungsvorrichtung erst nach der Herstellung der Vorrichtung mit dem Zwischenabschnitt zu verbinden.

In einer weiteren Ausführungsform ist die Kopplungsvorrichtung eine Kanüle, vorzugsweise eine Silikonkanüle. Die Kanüle kann dabei derart beschaffen sein, dass sie eine ausreichende Länge aufweist, um mit einer extrakorporalen Herzunterstützungspumpe verbunden zu sein. Dies kann insbesondere bedeuten, dass die Kanüle eine Länge zwischen 40 und 100 cm aufweisen kann. Bei dieser Ausführungsform kann es vorteilhaft sein, dass die Dauer einer Operation möglichst kurz gehalten werden kann und dass zusätzliche Verbindungsstücke zwischen einer Herzunterstützungspumpe und der Vorrichtung entfallen. Somit besteht eine höhere Wahrscheinlichkeit, dass sich keine Thromben bilden.

Silikon kann dabei ein bevorzugtes Material sein, um die Kanüle aufzubauen. Silikon ist biokompatibel und kann je nach Wandstärke gute Steifigkeitseigenschaften aufweisen. Insbesondere scheinen Silikonkanülen gut geeignet, dem Ansaugdruck einer Pumpe entgegenzuwirken, ohne dass das Innere der Kanüle kollabiert. Jedoch kann die Kanüle auch aus anderen biokompatiblen Materialien bestehen.

In einer weiteren Ausführungsform ist die Kopplungsvorrichtung ein Nahtring. In dieser Ausführungsform kann ein Nahtring, wie er beispielsweise bei intrakorporalen Herzunterstützungssystemen am Apex des Herzens eingesetzt wird, an der Vorrichtung im Bereich der dritten Öffnung bzw. die dritte Öffnung umlaufend angeordnet werden. Die Blutpumpe kann dann mit ihrem Einlass mit dem Nahtring verbunden werden und so eine fluiddichte Verbindung zwischen dem Herzunterstützungssystem und der Vorrichtung hergestellt werden. Dabei kann die Blutpumpe direkt in den Nahtring gesteckt werden oder über eine Kanüle mit der Vorrichtung verbunden werden.

In einer weiteren Ausführungsform umfasst die Vorrichtung als Kopplungsvorrichtung einen Adapter zum Anschluss einer Kanüle bzw. eines Nahtrings an eine Blutpumpe. Dabei kann der Adapter derart beschaffen sein, dass eine gängige Kanüle für ein extrakorporales System auf den Adapter aufgesteckt werden kann.

Beispielsweise kann hier ähnlich einem Luer-Verschlusssystem gearbeitet werden.

In einer weiteren Ausführungsform sind der erste und der zweite Endabschnitt so ausgebildet, dass sie mit einem Blutgefäß vernähbar oder konnektierbar, d.h. fluiddicht verbindbar sind. Zur Konnektierung kann beispielsweise ein Konnektor mittels Fadenschnürung, Kabelbinder, oder einer Manschette vorgesehen sein. Der erste und der zweite Endabschnitt können somit aus anderen Materialien als der Zwischenabschnitt bestehen. Beispielsweise kann ein Material des ersten bzw. des zweiten Endabschnitts so gewählt sein, dass es eine dünnere Wandstärke hat und somit einfacher mit einem Gefäß vernäht oder auf andere Weise verbunden werden kann. Beispielhafte Materialien sind Dacron® (DuPont), ein alternatives Polyethylenglykolterephtarat oder ePTFE (expandiertes Polytethraflourethylen). Im Falle einer fluiddichten Verbindung mittels einer Konnektierung kann der erste und/oder zweite Endabschnitt auch ein Metall oder biokompatiblen Kunststoff umfassen, um dem zur Konnektierung gewählten Konnektor eine Gegenkraft zu bieten, so dass das zwischen dem Konnektor und dem ersten oder zweiten Endabschnitt liegende Ende des Blutgefäß fest mit der Vorrichtung zu koppeln

In einer weiteren Ausführungsform ist der Hohlkörper einstückig ausgebildet, d. h., dass der erste und der zweite Endabschnitt und der Zwischenabschnitt aus dem gleichen Material bestehen. Dies kann beispielsweise die Herstellung der Vorrichtung vereinfachen und eventuelle Thrombenbildung im Bereich der Übergänge zwischen Endabschnitt und Zwischenabschnitt vermeiden.

In einer weiteren Ausführungsform besitzt der Hohlkörper einen Querschnitt, der vom ersten Endabschnitt zum Zwischenabschnitt zunimmt und vom Zwischenabschnitt zum zweiten Endabschnitt wieder abnimmt. Diese sogenannte bauchige Ausführungsform des Hohlkörpers bewirkt, dass das durch das erste und das zweite Blutgefäß einströmende Blut ein vergrößertes Volumen vorfindet, bevor es durch die dritte Öffnung in den Einlass einer Herzpumpe gesogen wird. Dies entlastet die mit der Vorrichtung verbundenen Gefäße zusätzlich. Des Weiteren kann durch eine Änderung der Wanddicke in manchen Bereichen des Hohlkörpers, beispielsweise im Zwischenbereich, eine leichtere Dehnbarkeit des Hohlkörpers in diesen Bereichen erreicht werden ("Compliance").

In einer weiteren Ausführungsform ist die bauchige Ausführungsform des Hohlkörpers asymmetrisch ausgebildet. Dies kann bedeuten, dass der Querschnitt nicht in alle Richtungen um eine Körperachse des Hohlkörpers zunimmt, sondern lediglich in den Richtungen eines Winkels um weniger oder gleich 180° um die Körperachse herum. In dieser Ausführungsform kann die bauchige Hälfte des Hohlkörpers derart im Körper angeordnet werden, dass der Bauch am Platz des beispielsweisen rechten Atriums zu liegen kommt.

In einer weiteren Ausführungsform ist der Hohlkörper so beschaffen, dass er eine Länge zwischen dem ersten und dem zweiten Endabschnitt zwischen 3 und 15 cm aufweist. Auf diese Weise wird den Gegebenheiten innerhalb des menschlichen Körpers Rechnung getragen, aber zugleich gewährleistet, dass die Pumpe sicher mit der Kopplungsvorrichtung verbunden werden, ohne zusätzlichen Stress auf die Blutgefäßenden auszuüben.

Das hier vorgestellte System bzw. die Vorrichtung kann dazu verwendet werden, bei einem Failing-Fontan-Patienten eine Herzentlastung vor einer Herztransplantation sicherzustellen, was dazu führt, dass das Gefäßsystem des Patienten besser auf die bevorstehende Transplantation vorbereitet ist.

Nachstehend soll die Erfindung anhand einiger Figuren näher erläutert werden. Es zeigt
- Fig. 1: einen Ausgangszustand vor einer Ausführungsform des Verfahrens zur Implantation einer Vorrichtung;
- Fig. 2: eine bereits vorimplantierte Vorrichtung mit schematisch dargestellten Pumpenanschlüssen;
- Fign. 3A und 3B: eine erste Ausführungsform einer Vorrichtung;
- Fign. 4A und 4B: eine weitere Ausführungsform einer Vorrichtung;
- Fign. 5A und 5B: eine weitere Ausführungsform einer Vorrichtung;
- Fig. 6: ein System mit einer Vorrichtung nach den Figuren 5;
- Fign. 7A und 7B: eine weitere Ausführungsform einer Vorrichtung; und
- Fig. 8: eine Darstellung der Verbindung der Kanülen mit dem körpereigenen Herz-/Gefäßsystem.

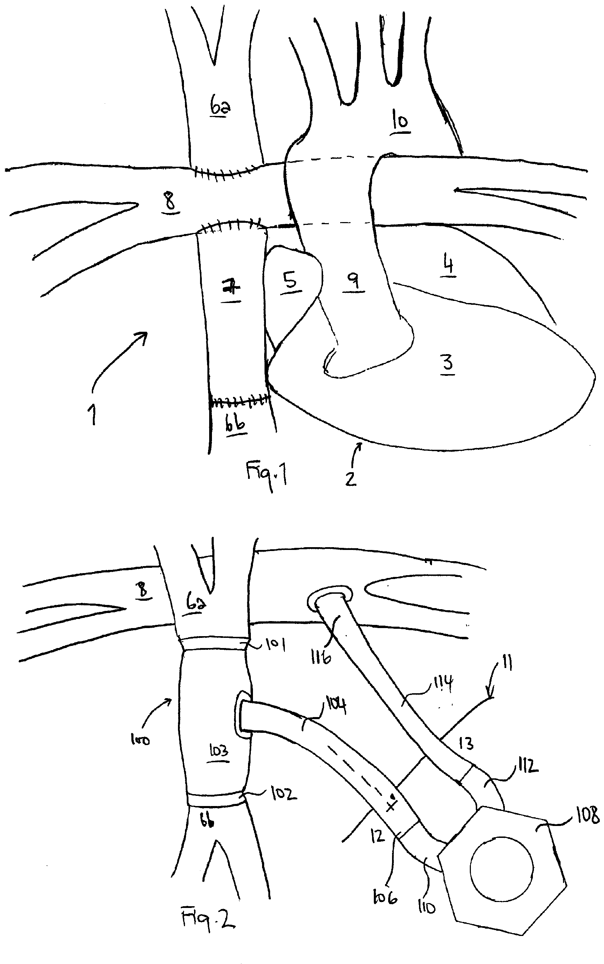

Anhand der Figur 1 soll eine mögliche Ausgangsposition vor der Implantation der anmeldungsgemäßen Vorrichtung dargestellt werden. Es handelt sich hierbei um die von Marceletti im Jahr 1988 vorgeschlagene cavo-pulmonale Anastomose, bei der die Verbindung zwischen der oberen und der unteren Vena cava zum rechten Vorhof hin getrennt wurde. In Figur 1 ist ein Ausschnitt des Herzkreislaufsystems 1 mit einem Herz 2 dargestellt, das lediglich ein Ventrikel 3, einen linken Vorhof 4 sowie einen rechten Vorhof 5 aufweist. Die Herzklappe zwischen dem Ventrikel 3 und dem rechten Vorhof 5 ist dauerhaft verschlossen worden, so dass das Ventrikel lediglich eine Verbindung zum linken Vorhof hat. Die obere Vena cava (SVC) 6a und ein Fontantunnel 7 wurden mittels einer Naht mit der Pulmonalarterie 8 verbunden. Der Fontantunnel 7 wurde an seinem unteren Ende mit der unteren Vena cava (IVC) 6b vernäht und kann beispielsweise eine aus PTFE gefertigte Prothese sein. Somit strömt Blut passiv von den Gefäßen 6a, 6b und 7 in die Pulmonalarterien durch die Lungen und anschließend in den linken Vorhof 4. Vom linken Vorhof 4 wird das Blut in das Ventrikel 3 transportiert und anschließend über die Aorta 9 in den Aortenbogen 10 und von dort in die verschiedenen Körperarterien transportiert.

Alternativ können die untere und die obere Hohlvene auch weiterhin mit dem rechten Vorhof verbunden sein, wobei die Herzklappe zum Ventrikel geschlossen ist und der Eingang zur Pulmonalarterie in den rechten Vorhof verlegt wird. Auch durch ein derartiges Vorgehen wird ein passiver Kreislauf zu den Lungen hin geschaffen.

Ausgehend von dem Ausführungsbeispiel der Figur 1 kann nun eine anmeldungsgemäße Vorrichtung mit dem Herzkreislaufsystem 1 verbunden werden.

Hierzu werden die SVC 6a und der Fontantunnel 7 von der Pulmonalarterie 8 getrennt, der Fontantunnel entfernt oder zurückgeschnitten, so dass ein Rand des Fontantunnels zum Verbinden der Vorrichtung verbleibt (beispielsweise mittels Vernähend er Vorrichtung mit dem verbleibenden Rand des Fontantunnels oder durch Konnektieren der Vorrichtung mit dem verbleibenden Rand des Fontantunnels) und anschließend, wie in Figur 2 gezeigt, eine Vorrichtung mit den Enden der SVC 6a und der IVC 6b vernäht (in der Ausführungsform des Verfahren, welches zum dargestellten Zustand der Figur 2 führt, wurde der Fontantunnel entfernt). Durch die Vorrichtung kann nun Blut von der SVC 6a und der IVC 6b von oben bzw. unten in die Vorrichtung strömen. Deren Hohlkörper 100 weist neben den mit den Gefäßen vernähten Endabschnitten 101 bzw. 102 einen Zwischenabschnitt 103 auf, der eine Kopplungsvorrichtung 104 in Form einer Kanüle integral umfasst. Die Kanüle wird dabei aus dem Körper des Patienten herausgeführt und durchstößt die Haut 11 am Punkt 12, beispielsweise unterhalb der Rippenbögen. Das Ende 106 der Kanüle 104, welche als Kopplungsvorrichtung dient, wird mit einer Blutpumpe 108 verbunden. Dabei wird der Einlass 110 der Blutpumpe 108 mit der Kanüle 104 über deren Ende 106 gekoppelt. Der Auslass 112 der Pumpe wird mit einer weiteren Kanüle 114 verbunden, wobei das nicht mit der Pumpe verbundene Ende der Kanüle 114 direkt an die Pulmonalarterie angeschlossen wird. Dazu kann das Ende 116 der Kanüle 114 beispielsweise mit der Pulmonalarterie 8 vernäht werden. Auf diese Weise wird eine aktive Versorgung (mittels der Blutpumpe 108) der Pulmonalarterie mit Blut aus der SVC 6a und der IVC 6b gewährleistet und somit das (in Figur 2 nicht mehr dargestellte) Herz 2 stark entlastet. Insbesondere können sich auch die Gefäße, wie die SVC bzw. die IVC, erholen, bevor ein Spenderherz eingesetzt wird.

Verschiedene Ausführungsformen der Vorrichtung 100 sollen nun anhand der weiteren Figuren erläutert werden.

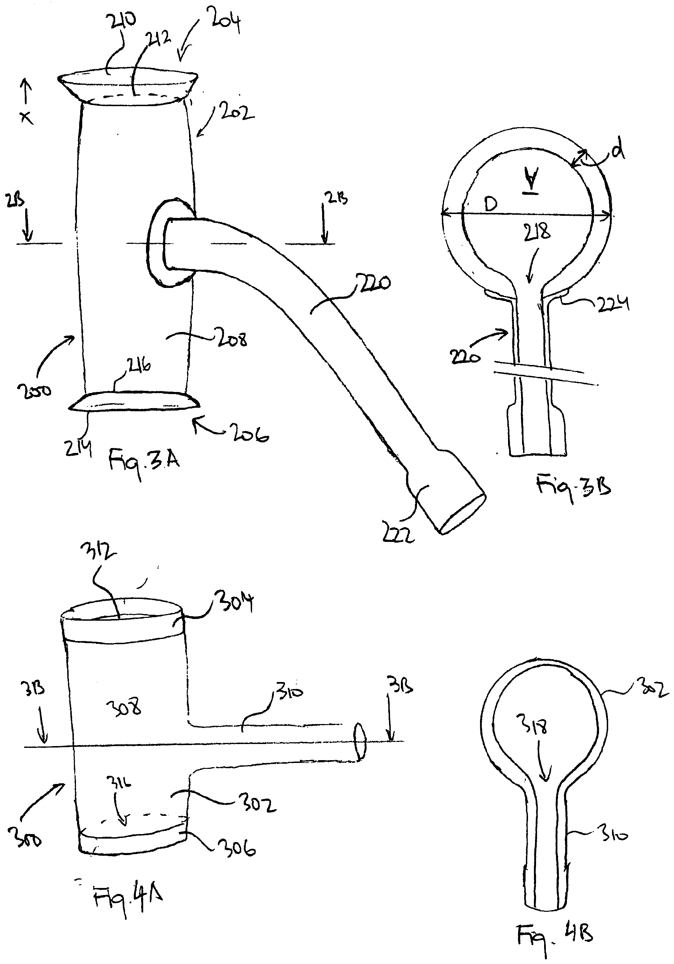

In Figur 3A ist eine Ausführungsform der Vorrichtung in der Längsaufsicht und in Figur 3B im Querschnitt gezeigt. Die Vorrichtung 200 umfasst einen Hohlkörper 202, der eine Länge L = 8 cm in x-Richtung und einen Durchmesser D = 3 cm aufweist. Dabei nimmt der Durchmesser vom ersten Endabschnitt 204 zum zweiten Endabschnitt 206 zunächst zu, erreicht ein Maximum in der Mitte des Zwischenabschnitts 208 und nimmt anschließend wieder ab. Somit hat der Hohlkörper eine bauchige Form. Der Hohlkörper an sich ist aus Silikon gefertigt. Dieser hat eine Wandstärke d von 3 mm. Diese Wandstärke bewirkt, dass die Kanüle bzw. der Hohlkörper 202 auf einfache Druckbelastungen unverformbar reagiert und somit sichergestellt ist, dass das innerhalb des Hohlkörpers vorhandene Volumen V als Blutreservoir zur Verfügung steht.

Der erste und der zweite Endabschnitt 204, 206 sind ähnlich aufgebaut. Daher werden nachstehend die Komponenten des ersten Endabschnitts stellvertretend auch für den zweiten Abschnitt erläutert.

Der erste Endabschnitt 204 umfasst einen Kragen 210, der aus einem gedünnten Silikon besteht, das beispielsweise mit einem künstlichen Gewebe vernäht werden kann. Dieses künstliche Gewebe kann anschließend mit der SVC vernäht werden. Durch das zusätzliche künstliche Gewebe können unterschiedliche Längen bei der Verbindung der SVC und der IVC berücksichtigt werden. Innerhalb des ersten Endabschnitts liegt eine erste Öffnung 212, durch die Blut in den Zwischenabschnitt 208 strömen kann. Analog zum Kragen 210 weist der zweite Endabschnitt einen Kragen 214 und eine zweite Öffnung 216 auf. Das Material des Zwischenabschnitts 208 ist so gewählt, dass dessen Wandstärke größer ist als die Wandstärke des Materials im Bereich des ersten bzw. zweiten Endabschnitts. Hierdurch wird gewährleistet, dass der Zwischenabschnitt steifer als der erste und zweite Endabschnitt ist.

Im Querschnitt der Figur 3B betrachtet ist erkennbar, dass der Hohlkörper 202 ferner eine dritte Öffnung 218 umfasst und im Bereich der Öffnung 218 eine Kopplungsvorrichtung 220 vorhanden ist, die im vorliegenden Ausführungsbeispiel als Silikonkanüle ausgebildet ist. Die Silikonkanüle 220 ist dabei mit dem Hohlkörper 202 stoffschlüssig verbunden. Beispielsweise kann die Verbindung mittels Klebstoff oder mittels Schweißen realisiert werden. Aber auch eine kraftschlüssige Verbindung, beispielsweise durch Vernähen oder Tackern, wäre möglich. Die Silikonkanüle 220 hat eine Länge von 40 cm und ist so ausgeführt, dass das vom Hohlkörper 202 wegführende Ende 222 direkt mit einer extrakorporalen Blutpumpe, wie beispielsweise der Blutpumpe 108 der Figur 2, verbunden werden kann. Der Durchmesser der Kanüle 220 kann so gewählt werden, dass er für den entsprechenden Einsatzzweck geeignet ist. Das heißt, dass bei kleineren Kindern beispielsweise eine Kanüle mit dünnerem Durchmesser, bei größeren Kindern eine Kanüle mit größerem Durchmesser gewählt werden kann.

Die Kanüle 220 besitzt auf der Außenseite des Hohlkörpers 202 einen Flansch 224, der als Verstärkung der Verbindung mit dem Hohlkörper 202 dient. Hierdurch wird eine bessere Befestigung zwischen der Kanüle 220 und dem Hohlkörper 202 gewährleistet.

Eine weitere Ausführungsform einer Vorrichtung findet sich in den Figuren 4. Die Vorrichtung 300 der Figur 4A ist in Figur 4B im Querschnitt dargestellt. Die Vorrichtung 300 umfasst einen Hohlkörper 302, der beispielsweise aus einem Silikon mit gleichbleibendem Querschnittsdurchmesser besteht. Der Hohlkörper 302 umfasst ferner einen ersten Endabschnitt 304 und einen zweiten Endabschnitt 306, für die ein gewebeartiges, jedoch fluiddichtes Material gewählt wurde. Zwischen dem ersten Endabschnitt 304 und dem zweiten Endabschnitt 306 befindet sich ein Zwischenabschnitt 308, in den eine Kanüle 310 direkt integriert ist. Der Zwischenabschnitt 308 und die Kanüle 310 sind integral miteinander verbunden und aus einem Stück gefertigt. Der erste Endabschnitt 304 und der zweite Endabschnitt 306 sind mit dem Zwischenabschnitt 308 verschweißt und können direkt mit Gefäßenden beispielsweise vernäht oder vertackert werden. Am zum Zwischenabschnitt 308 hin gelegenen Ende des ersten Endabschnitts 304 liegt eine Öffnung 312, die die erste Öffnung ist. Eine entsprechende zweite Öffnung ist schematisch dargestellt und mit dem Bezugszeichen 316 versehen. In Figur 4B ist erkennbar, dass sich die Kanüle 310 direkt an den Hohlkörper 302 anschließt, wobei das Lumen der Kanüle 310 im Bereich der dritten Öffnung 318 in den Hohlraum des Hohlkörpers 302 mündet.

Die in den Figuren 4 gezeigte geometrische Form des Hohlkörpers als gleichbleibender Durchmesser kann beispielsweise auch in der Ausführungsform der Figur 3A verwendet werden. Ebenfalls kann die Ausführung der Endabschnitte als Gewebematerialien, die mit dem Zwischenabschnitt 308 verschweißt sind, auch in der in den Figuren 3 dargestellten Vorrichtung verwendet werden, so wie die Endabschnitte aus den Figuren 3 auch in der Ausführungsform nach den Figuren 4 verwendet werden können.

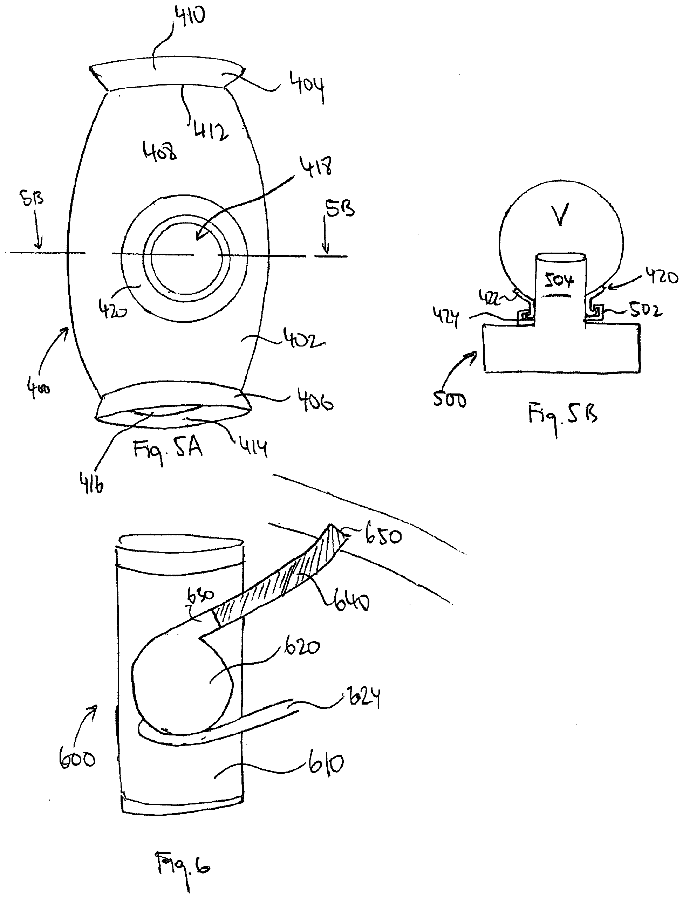

Eine weitere Ausführungsform einer Vorrichtung ist in den Figuren 5 dargestellt. Dabei ist Figur 5B ein Querschnitt im Bereich der dritten Öffnung der in Figur 5A dargestellten Vorrichtung. Die Vorrichtung 400 umfasst, wie in den vorhergehenden Ausführungsbeispielen, einen Hohlkörper 402, der über einen ersten Endabschnitt 404 und einen zweiten Endabschnitt 406 verfügt. Zwischen diesen beiden Endabschnitten befindet sich ein Zwischenabschnitt 408. Im vorliegenden Ausführungsbeispiel kann der Zwischenabschnitt 408 aus einem biokompatiblen Kunststoff oder einem Silikon gefertigt sein. Der erste und der zweite Endabschnitt 404, 406 sind mit dem Zwischenabschnitt 408 verschweißt und umfassen ein mit einem Gefäßende vernähbares Material. Auf diese Weise können die Gefäßenden, wie beispielsweise die Gefäßenden der SVC oder IVC, direkt mit der Vorrichtung vernäht werden. Im Zwischenabschnitt 408 ist zudem ein "Nahtring" angeordnet, der entweder direkt mit einer Pumpe gekoppelt werden kann oder an den eine Kanüle angeschlossen werden kann, die wiederum mit einer Blutpumpe gekoppelt werden kann. Der Nahtring stellt somit lediglich eine spezielle Ausführungsform eines Adapters dar, mit dem die Vorrichtung direkt mit einer Blutpumpe gekoppelt werden kann oder indirekt über eine weitere Kanüle mit einer Blutpumpe gekoppelt werden kann.

Der erste Endabschnitt umfasst neben dem Kragen 410 eine Öffnung 412. Analog hierzu umfasst der zweite Endabschnitt einen Kragen 414 und eine Öffnung 416. Der Kragen 410 bzw. 414 weist hierbei eine Breite von 2 cm auf und kann nach der Befestigung des Kragens mit dem Gefäßende gekürzt werden. Hierdurch wird dem Operateur die Möglichkeit gegeben, die Endabschnitte erst beim eigentlichen Implantieren der Vorrichtung zurechtzuschneiden, ohne die Vorrichtung mit zusätzlichen Abschnitten an das Gefäß anstückeln zu müssen.

Der Zwischenabschnitt 408 umfasst eine dritte Öffnung 418, die durch den Nahtring 420 umlaufen wird. In Figur 5B ist der Nahtring 420 im Querschnitt dargestellt. Der Nahtring 420 umfasst einen Kragen 422 und einen weiteren, durch eine Pumpe zu hintergreifenden Kragen 424, der vom Hohlkörper radial nach außen zeigt. Dieser "Nahtring" kann, nachdem die Vorrichtung mit einem Loch versehen wurde, nachträglich aufgebracht werden und mit dem Hohlkörper entweder verschweißt oder verklebt oder mittels einer Naht verbunden werden. Alternativ kann der Nahtring auch als Adapter ausgebildet werden, auf den eine Kanüle, wie beispielsweise die Kanüle 114 bzw. 104, aufgesteckt werden kann. Hierzu kann der Adapter beispielsweise vom Zwischenabschnitt radial hinweg zeigend einen männlichen Teil eines Luer-Schlosses aufweisen, auf den eine Kanüle mit einem weiblichen Luer-Schloss aufgebracht werden kann. Jedoch sind auch andere im klinischen Bereich gängige Stecksysteme zum Verbinden eines Adapters mit einer Kanüle zum Einsatz mit der Vorrichtung möglich.

In der hier dargestellten Ausführungsform ist der Nahtring 420 so ausgestaltet, dass er durch eine Pumpe 500 hintergriffen werden kann. Hierzu kann die Pumpe beispielsweise einen Mechanismus 502 umfassen, der ein dauerhaftes Hintergreifen des Kragens 424 ermöglicht. Der Einlass 504 der Pumpe wird, wie in Figur 5B dargestellt, direkt in die dritte Öffnung 418 gesteckt, so dass der Eingang des Einlasses 504 im Volumen V des Hohlkörpers 402 zu liegen kommt. Strömt nun Blut von der SVC und der IVC in den Zwischenabschnitt, wird dieses durch den Einlass 504 der Pumpe in die Pumpe gezogen, mit einer Kraft beaufschlagt und durch den Auslass 506 ausgestoßen. Dabei kann es sich bei dieser intrakorporalen Herzunterstützungspumpe beispielsweise um ein Axialsystem zum Beaufschlagen des Blutes mit einer axialen Kraft oder um ein Radialsystem zum Beaufschlagen des Blutes mit einer Radialkraft handeln. Auch kann die Vorrichtung zunächst nur mit einer Öffnung 418 versehen werden und je nach Anwendung erst kurz vor dem Eingriff mit einem entsprechenden Adapter ausgestattet werden. Dabei kann auf die mit Herzunterstützungssystemen gelieferten Adapter, wie beispielsweise deren Nahtringe, zurückgegriffen werden.

Anhand der Figur 6 soll ein System 600 aus einer Vorrichtung 610 und einer Blutpumpe 620 illustriert werden. Die Blutpumpe 620 wird dabei mit der Vorrichtung 610 wie in Figur 5B gezeigt gekoppelt. Die Blutpumpe 620 verfügt über einen Antrieb, der mittels des Kabels 624 mit Energie versorgt wird. Dabei kann das Kabel zu einem transkutan implantierten Energieversorgungssystem geführt sein oder durch die Haut des Patienten nach außen geführt werden, um dort mit einer Energiequelle verbunden zu werden. Der Auslass 630 der Pumpe ist mit einem Auslassgraft 640 verbunden, der an seinem dem Auslass 630 gegenüberliegenden Ende 650 mit der Pulmonalarterie gekoppelt wird. Hierbei kann beispielsweise auf einen bestehenden Fontan-Graft oder Fontan-Tunnel zurückgegriffen werden und dieser mit dem Auslass der Pumpe gekoppelt werden.

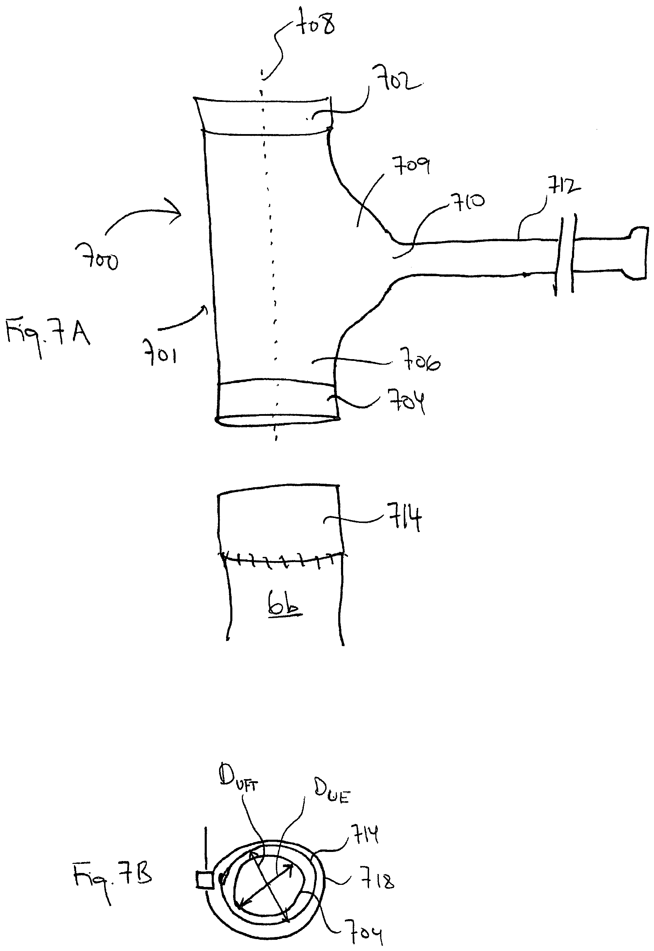

Anhand der Figuren 7A und 7B soll eine weitere Ausführungsform einer Vorrichtung und eines anmeldungsgemäßen Verfahren illustriert bzw. erläutert werden.

Die Vorrichtung 700 umfasst einen Hohlkörper 701 mit einem oberen Endabschnitt 702, einem unteren Endabschnitt 704 und einen dazwischenliegenden Zwischenabschnitt 706. Der Zwischenabschnitt weist einen asymmetrischen Querschnitt auf, wobei die rechts neben der Körperachse 708 liegende Fläche vom oberen Endabschnitt zum unteren Endabschnitt zunächst anwächst und wieder abnimmt, wohingegen die links der Körperachse liegende Fläche im Wesentlichen konstant bleibt. Dieser bauchige Abschnitt 709 ist also nicht auf beiden Seiten der Körperachse 708 ausgeprägt, sondern lediglich auf einer Seite. Allgemein kann unter einem asymmetrischen Querschnitt ein Querschnitt verstanden werden, welcher entlang einer Körperachse zumindest über einen Winkelbereich zwischen 30° und 180° einen konstanten Radius aufweist.

Zudem kann die Wandstärke des Hohlkörpers im Bereich des größten Querschnitts, d.h. im Bereich der dritten Öffnung 710 an welche sich die Kopplungsvorrichtung 712 anschließt, reduziert sein, um eine bessere Compliance, beispielsweise im Bereich des rechten Vorhofs zu erreichen. Alternativ zur Kopplungsvorrichtung kann die dritte Öffnung auch einen Konnektor für eine Blutpumpe umfassen.

Die IVC 6b ist weiterhin mit einem unteren Rand 714 des Fontantunnels versehen, d.h. der Fontantunnel wurde nicht komplett entfernt, sondern der Rand stehen gelassen, um keine weitere Naht in die IVC einbringen zu müssen und somit den Patienten weiter zu entlasten. Obgleich in den Figuren nicht dargestellt, kann - alternativ oder zusätzlich zum unteren Rand - ein oberer Rand des Fontantunnels, welcher mit der Pulmonalarterie vernäht ist, im Körper verbleiben. Auf diese Weise wird auch die Anbindung der Vorrichtung an die Pulmonalarterie bzw. den oberen Rand des Fontantunnels vereinfacht. Dazu wird der Fontantunnel 7 derart durchgetrennt, dass ein oberer und/oder unterer Rand, beispielsweise mit einer Breite zwischen 0,5 cm und 3 cm, mit der IVC bzw. Pulmonalarterie verbunden bleiben, so dass keine neuen zusätzlichen Nähte im körpereigenen Gewebe gesetzt werden müssen.

Im vorliegenden Beispiel ist der Hohlkörper 701 am oberen Endabschnitt und im Zwischenabschnitt 706 aus einem Silikon gefertigt. Der untere Endabschnitt 704 weist neben dem Silikon eine metallische Verstärkung auf, welcher auf der Außenseite 716 des Hohlkörpers angeordnet ist. Die metallische Verstärkung kann beispielsweise gesintertes Titan sein, welches die Einwachseigenschaften des unteren Endabschnitts verbessert. Der Durchmesser D_{UE} des unteren Endabschnitts ist im vorliegenden Ausführungsbeispiel kleiner als der Durchmesser D_{UFT} des unteren Rands 714 des Fontantunnels 7. Auf diese Weise kann der untere Endabschnitt in den unteren Rand des Fontantunnels eingeführt werden und anschließend mit einem Verbindungsmittel, wie z.B. einem Kabelbinder 718, fluiddicht verbunden werden.

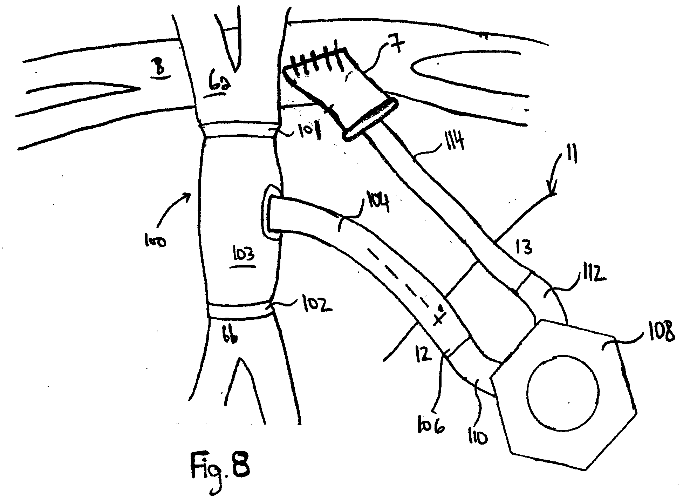

Anhand der Figur 8 wird das bereits erwähnte Verfahren illustriert, bei welcher der Fontantunnel 7 - anders als in der Fig. 2 dargestellt - nicht entfernt wird, sondern in Gänze oder gekürzt mit der weiteren Kanüle 114 verbunden wird. Bei dieser Ausführungsform muss die Kanüle 114 nicht direkt mit der Pulmonalarterie 8 verbunden werden, so dass bei dem hier vorgeschlagenen Verfahren die Pulmonalarterie nicht weiter belastet wird.

Die hier vorgeschlagene Vorrichtung kann beispielsweise mittels eines Gießverfahrens geformt werden. Es ist jedoch auch möglich, beispielsweise den Zwischenabschnitt oder den gesamten Hohlkörper durch ein dreidimensionales Druckverfahren herzustellen. Auf diese Weise kann besonders effizient auf die individuellen Gegebenheiten innerhalb des Patientenkörpers Rücksicht genommen werden.

Obgleich bislang das Pumpensystem nur als Übergangssystem vor einer Herztransplantation beschrieben wurde, kann das Pumpensystem auch als dauerhaftes Herzunterstützungssystem verwendet werden, sofern kein geeignetes Spenderherz zur Verfügung steht. In diesem Fall verbleiben die Pumpe oder die Kanülen zum Anschluss der Pumpe dauerhaft im Körper des Patienten.

## Patentansprüche

1. Vorrichtung (100; 200) zur Verbindung zweier Blutgefäßabschnitte, wobei die Vorrichtung einen fluiddichten Hohlkörper (202) mit einem ersten Endabschnitt (204), einem zweiten Endabschnitt (206) und einem zwischen dem ersten und zweiten Endabschnitt angeordneten Zwischenabschnitt (208) umfasst, wobei der erste Endabschnitt eine erste Öffnung (212), der zweite Endabschnitt eine zweite Öffnung (216) und der Zwischenabschnitt eine dritte Öffnung (218) aufweist und die erste und zweite Öffnung mit jeweils einem Gefäßabschnitt verbindbar sind und der Zwischenabschnitt im Bereich der dritten Öffnung eine Kopplungsvorrichtung (220; 310; 420) für eine Blutpumpe oder eine blutführende Kanüle aufweist.

2. Vorrichtung nach Anspruch 1, wobei die Kopplungsvorrichtung integral mit dem Zwischenabschnitt verbunden ist.

3. Vorrichtung nach einem der Ansprüche 1 oder 2, wobei die Kopplungsvorrichtung eine Kanüle, vorzugsweise eine Silikonkanüle, ist.

4. Vorrichtung nach einem der Ansprüche 1 oder 2, wobei die Kopplungsvorrichtung ein Nahtring ist.

5. Vorrichtung nach einem der vorhergehenden Ansprüche, wobei die Kopplungsvorrichtung ein Adapter zum Anschluss einer Pumpe oder einer blutführenden Kanüle ist.

6. Vorrichtung nach einem der vorhergehenden Ansprüche, wobei der erste und der zweite Endabschnitt derart ausgebildet sind, dass sie mit einem Blutgefäß fluiddicht verbindbar sind.

7. Vorrichtung nach einem der vorhergehenden Ansprüche, wobei der Hohlkörper einstückig ist.

8. Vorrichtung nach einem der vorhergehenden Ansprüche, wobei der Hohlkörper einen Querschnitt besitzt, welcher vom ersten Endabschnitt zum Zwischenabschnitt zunimmt und vom Zwischenabschnitt zum zweiten Endabschnitt wieder abnimmt.

9. Vorrichtung nach einem der vorhergehenden Ansprüche, wobei der Hohlkörper eine Länge zwischen dem ersten und zweiten Endabschnitt zwischen 2 und 15 cm besitzt.

10. Vorrichtung nach einem der vorhergehenden Ansprüche, wobei die zu verbindenden Blutgefäßabschnitte die Vena cava superior und die Vena cava inferior sind.

11. System(600), umfassend eine Blutpumpe (108; 500; 620) sowie eine Vorrichtung nach einem der vorhergehenden Ansprüche.

12. System nach dem vorhergehenden Anspruch, wobei die Blutpumpe eine extrakorporale oder intrakorporale Herzunterstützungspumpe ist.

13. Verwendung der Vorrichtung nach einem der Ansprüche 1 bis 10 zur Herzentlastung eines Failing-Fontan-Patienten.

14. Verfahren zur Implantation einer Vorrichtung nach einem der Ansprüche 1 bis 10, wobei der erste Endabschnitt mit der Vena cava superior und der zweite Endabschnitt mit der Vena cava inferior verbunden wird, die Kopplungsvorrichtung mit einem Eingang einer Blutpumpe gekoppelt wird und ein Ausgang der Blutpumpe mit einer Pulmonararterie verbunden wird.

15. Verfahren nach dem vorhergehenden Anspruch, wobei die Blutpumpe eine extrakorporale Herzpumpe ist und mit einer sich zwischen der dritten Öffnung und dem Eingang der Blutpumpe aus einem Körper erstreckenden Kanüle verbunden wird und der Ausgang der Blutpumpe mittels einer weiteren, von einer Pulmonararterie sich zum Ausgang erstreckenden Kanüle gekoppelt wird.

16. Verfahren nach Anspruch 14, wobei die Blutpumpe eine intrakorporale Herzpumpe und die Kopplungsvorrichtung ein Kopplungsring ist, welcher fest mit dem Hohlkörper verbunden ist.
